# EUROPEAN PATENT SPECIFICATION

(11) **EP 4 190 234 B1**
(45) Date of publication and mention of the grant of the patent: **31.12.2025**
(21) Application number: 22794950.0
(22) Date of filing: 27.04.2022
(51) Int. Cl.: A61B 5/02, A61B 5/024, A61B 5/08

(54) **WEARABLE DEVICE BASED ON PHOTOPLETHYSMOGRAPHY (PPG) AND CONTROL METHOD THEREFOR**
TRAGBARE VORRICHTUNG AUF DER BASIS VON PHOTOPLETHYSMOGRAFIE (PPG) UND STEUERUNGSVERFAHREN DAFÜR
DISPOSITIF PORTABLE BASÉ SUR LA PHOTOPLÉTHYSMOGRAPHIE (PPG) ET SON PROCÉDÉ DE COMMANDE

(30) Priority: 30.04.2021 CN 202110484620
(43) Date of publication of application: 07.06.2023
(73) Proprietor: Honor Device Co., Ltd., Shenzhen, Guangdong 518040 (CN)
(72) Inventor: CAI, Xinpei, Shenzhen, Guangdong 518040 (CN); TAN, Yinjiong, Shenzhen, Guangdong 518040 (CN); LIU, Yi, Shenzhen, Guangdong 518040 (CN)
(74) Representative: Gill Jennings & Every LLP
(86) International application number: PCT/CN2022/089654
(87) International publication number: WO 2022/228480

(56) References cited:
- WO-A1-2020/088639
- CN-A- 108 430 322
- CN-A- 109 645 972
- CN-A- 110 087 543
- CN-A- 110 179 438
- CN-A- 110 432 883
- CN-A- 110 970 410
- CN-A- 111 132 610
- CN-A- 111 329 463
- CN-A- 111 973 167
- CN-A- 111 973 167
- CN-A- 112 190 248
- CN-A- 112 190 248
- US-A1- 2016 058 312
- US-A1- 2018 146 870
- US-A1- 2021 007 634

## Description

This application claims priority to Chinese Patent Application No. 202110484620.7, filed with the China National Intellectual Property Administration on April 30, 2021 and entitled "WEARABLE DEVICE BASED ON PHOTOPLETHYSMOGRAPHY PPG AND CONTROL METHOD THEREOF".

### TECHNICAL FIELD

This application relates to the technical field of terminals, and in particular, to a wearable device based on photoplethysmography PPG and a control method thereof.

### BACKGROUND

Currently, with development of terminal technologies, terminal devices have become a part of people's work and life. To meet the needs of users for health management, many terminal devices can provide the users with a human body data monitoring function. For example, a user may measure a human body feature such as a heart rate, a respiration rate, or blood oxygen of a human body by using a wearable device such as a smartwatch.

Generally, the terminal device may be configured with a photoplethysmography (photo plethysmo graphy, PPG) module configured to measure human body features, where the PPG module may include a photodiode (Photo diode, PD) and an LED (light emitting diode, LED). When the user monitors a human body feature by using the terminal device including the PPG module, a signal may be transmitted through the LED in the PPG module, a light signal reflected by human tissue through the PD is received, and then the human body feature is obtained based on the light signal.

However, in some scenarios, the foregoing mentioned smartwatch including the PPG module cannot obtain a stable light signal, and therefore cannot obtain an accurate human body feature based on the light signal.

CN110179438A relates to a built-in illuminating part encircling type signal detection structure and method for a PPG sensor. CN112190248A relates to a wearing state detection method and device and wearable equipment. CN111973167A relates to wearable equipment, an optical device, an optical module and a packaging method of the optical module. CN110432883A discloses a photoplethysmograph and a terminal. CN110087543Arelates to Systems, methods and devices for reducing noise in health monitoring including monitoring systems, methods and/or devices receiving a health signal and/or having at least one electrode or sensor for health monitoring. CN108430322A discloses a device, method and computer program product for continuous monitoring of vital signs. CN111329463A discloses a motion artifact eliminating system for measurement based on PPG heart rate and realizing method of motion artifact eliminating system for measurement based on PPG heart rate. WO2020088639A1 discloses a heart rate detection method and an electronic device. US2016058312A1 relates to a device that measures a photoplethysmographic signal.

### SUMMARY

Embodiments of this application provide a wearable device based on photoplethysmography PPG and a control method thereof, so that a valid PPG signal can be obtained based on an annular surrounding structure of a plurality of PDs in different scenarios, and then an accurate human body feature can be further obtained based on the signal. The invention is defined by the appended claims. Embodiments not falling within the scope of the claims are for illustration.

According to a first aspect, an embodiment of this application provides a wearable device based on photoplethysmography PPG, where the wearable device includes a PPG module and a processor; and the PPG module includes a plurality of light emitting diodes LEDs and a plurality of photodiodes PDs; the plurality of PDs are distributed around the plurality of LEDs in a surrounding structure; each of the LEDs is configured to emit light signals; and the LED is an LED in which red light, green light, and infrared light are combined, and the light signals include: a green light signal, a red light signal, and/or an infrared light signal; each PD is configured to: receive the light signals, and transmit the light signals to the processor; and the processor is configured to obtain a heart rate feature, a blood oxygen feature, and/or a respiration rate feature based on the light signals received from the plurality of PDs.

Specifically, centers of the plurality of LEDs may coincide with centers of the plurality of PDs. In this way, the wearable device can obtain a valid PPG signal based on the annular surrounding structure of the plurality of PDs, and then can obtain an accurate human body feature based on the signal.

According to the invention, the processor is further configured to: control the LED to emit a green light signal, or a red light signal when it is detected that the light intensity is greater than or equal to a light intensity threshold; or control the LED to emit an infrared light signal when it is detected that the light intensity is less than the light intensity threshold. In this way, the wearable device can flexibly control the color of light emitted by the LED based on light intensity in different application scenarios.

According to the invention, the processor is further configured to control the frequency of the light signal emitted by the LED when it is detected that the wearable device is in a moving state. In this way, the wearable device can flexibly control the luminous intensity of the LED based on the light intensity in different application scenarios.

In an illustrative example, the light signals transmitted by each PD to the processor includes intensity of the light signal and an identifier of an LED that emits the light signal; where a light path relationship between each LED and each PD is provided in the processor, and the light path relationship includes: a near light path, a remote light path, and a medium-distance light path; the processor is further configured to: obtain a first light signal belonging to the near light path and the medium-distance light path from the light signals from the plurality of PDs, and obtain the heart rate feature based on the first light signal; and/or obtain a second light signal belonging to the remote light path and the medium-distance light path from the light signals from the plurality of PDs, and obtain the blood oxygen feature based on the second light signal. In this way, when obtaining data of a human body feature, the wearable device can avoid calculating light signals in all light paths, and can save a calculation overhead of the wearable device by obtaining the light signals in the light paths corresponding to the application scenarios.

In a possible implementation, the processor is further configured to: obtain a third light signal having the strongest signal intensity from the light signals from the plurality of PDs, and obtain the heart rate feature, the blood oxygen feature, and/or the respiration rate feature from the third light signal; or the processor is further configured to: obtain a fourth light signal by calculating an average value of the light signals from the plurality of PDs, and obtain the heart rate feature, the blood oxygen feature, and/or the respiration rate feature from the fourth light signal; or the processor is further configured to: obtain a fifth light signal by weighting calculation based on the light signals from the plurality of PDs and a weight of each PD, and obtain the heart rate feature, the blood oxygen feature, and/or the respiration rate feature based on the fifth light signal; where the weight of each PD is determined by the processor based on a quantity of times that the light signals emitted by each PD are used as an obtaining basis in a history record, or the weight of each PD is preset. In this way, the wearable device can accurately obtain a human body feature based on the foregoing described method by avoiding effects on obtaining the human body feature due to PD damage or the like or inaccurate reception of a PD signal.

In a possible implementation, the PPG module further includes fast charging pins; the fast charging pins are provided outside the plurality of PDs; and the fast charging pins are configured to provide a fast charging interface for the wearable device. In this way, long battery life of the wearable device is further ensured, and a service time of the wearable device is prolonged.

In a possible implementation, a quantity of the LEDs is two; a quantity of PDs is eight; and the eight PDs are arranged in an eight-equal distribution with a midpoint of a connecting line of the two LEDs as a circle center. In this way, the wearable device can obtain a valid PPG signal based on the surrounding structure formed by the eight PDs, and obtain an accurate human body feature based on the signal.

In a possible implementation, the structure of the PPG module is a concentric circular structure or a concentric square structure. Thus, regardless of the structure of the PPG module, the surrounding structure formed by the plurality of PDs in the PPG module can obtain a valid PPG signal, and the wearable device can obtain an accurate human body feature based on the signal.

According to a second aspect, an embodiment of this application provides an control method, applied to the wearable device according to any one of the first aspect, where the method includes: receiving light signals from a plurality of PDs; and obtaining a heart rate feature, a blood oxygen feature, and/or a respiration rate feature based on the light signals received from the plurality of PDs, where each PD is configured to receive light signals transmitted by an LED, and transmit the light signals to a processor.

According to the invention, the controlling a color of light emitted by the LED based on light intensity includes: controlling the LED to emit a green light signal, or a red light signal when it is detected that the light intensity is greater than or equal to a light intensity threshold; or controlling the LED to emit an infrared light signal when it is detected that the light intensity is less than the light intensity threshold.

According to the invention, the method further includes: controlling the frequency of the light signal emitted by the LED when it is detected that the wearable device is in a moving state.

In an illustrative example, each of the light signals transmitted by the PDs to the processor includes intensity of the light signal and an identifier of an LED that emits the light signal; and a light path relationship between each LED and each PD is provided in the processor, and the light path relationship includes: a near light path, a remote light path, and a medium-distance light path, and the method further includes: receiving the light signals that are transmitted by the PDs and that include intensity of the light signal and an identifier of an LED that emits the light signal; where a light path relationship between each LED and each PD is provided in the processor, and the light path relationship includes: a near light path, a remote light path, and a medium-distance light path; obtaining a first light signal belonging to the near light path and the medium-distance light path from the light signals from the plurality of PDs and obtaining the heart rate feature based on the first light signal; and/or obtaining a second light signal belonging to the remote light path and the medium-distance light path from the light signals from the plurality of PDs, and obtaining the blood oxygen feature based on the second light signal.

In a possible implementation, the obtaining a heart rate feature, a blood oxygen feature, and/or a respiration rate feature based on the light signals received from the plurality of PDs includes: obtaining a third light signal having the strongest signal intensity from the light signals from the plurality of PDs, and obtaining the heart rate feature, the blood oxygen feature, and/or the respiration rate feature from the third light signal; or obtaining a fourth light signal by calculating an average value of the light signals from the plurality of PDs, and obtaining the heart rate feature, the blood oxygen feature, and/or the respiration rate feature from the fourth light signal; or obtaining a fifth light signal by weighting calculation based on the light signals from the plurality of PDs and a weight of each PD, and obtaining the heart rate feature, the blood oxygen feature, and/or the respiration rate feature based on the fifth light signal; where the weight of each PD is determined by the processor based on a quantity of times that the light signals emitted by each PD are used as an obtaining basis in a history record, or the weight of each PD is preset.

According to a third aspect, an embodiment of this application provides a computer-readable storage medium. The computer-readable storage medium stores instructions, and when the instructions are executed, a computer is enabled to perform the control method according to any one of the second aspect or the implementations of the second aspect.

According to a fourth aspect, a computer program product is provided, includes a computer program. When the computer program is run, a computer is enabled to perform the control method according to any one of the second aspect or the implementations of the second aspect.

It should be understood that the second to fourth aspects of this application correspond to the technical solution according to the first aspect of this application, and beneficial effects obtained from the aspects and the corresponding feasible implementations are similar, and details are not described again.

### BRIEF DESCRIPTION OF DRAWINGS

FIG. 1 is a schematic diagram of a scenario according to an embodiment of this application;
FIG. 2 is a schematic diagram of an interface for recording a heart rate based on a smartwatch according to an embodiment of this application;
FIG. 3 is a schematic diagram of a principle of measuring a human body feature based on a PPG module according to an embodiment of this application;
FIG. 4 is a schematic diagram of a structure of a terminal device according to an embodiment of this application;
FIG. 5 is a schematic diagram of a structure of a smartwatch based on two LEDs+eight PDs+wired fast charging according to an embodiment of this application;
FIG. 6 is a schematic diagram of a structure of another smartwatch based on two LEDs+eight PDs+wired fast charging according to an embodiment of this application;
FIG. 7 is a schematic diagram of a structure of yet another smartwatch based on two LEDs+eight PDs+wired fast charging according to an embodiment of this application;
FIG. 8 is a schematic diagram of a structure of a smartwatch based on two LEDs+12 PDs+wired fast charging according to an embodiment of this application;
FIG. 9 is a schematic diagram of a structure of a smartwatch based on four LEDs+16 PDs+wired fast charging according to an embodiment of this application;
FIG. 10 is a schematic diagram of a structure of a smartwatch based on two LEDs+two PDs according to an embodiment of this application;
FIG. 11 is a schematic flowchart of a control method according to an embodiment of this application;
FIG. 12 is a schematic diagram of receiving a light signal through PPG in a moving state according to an embodiment of this application; and
FIG. 13 is a schematic diagram of a hardware structure of a control device according to an embodiment of this application.

### DESCRIPTION OF EMBODIMENTS

To clearly describe the technical solutions in embodiments of this application, in embodiments of this application, words such as "first" and "second" are used to distinguish between same items or similar items with basically the same functions and effects. For example, a first value and a second value are merely used to distinguish between different values, but not limit a sequence thereof. A person skilled in the art may understand that words such as "first" and "second" do not limit a quantity or an execution order, and the words such as "first" and "second" do not necessarily indicate a difference.

It should be noted that, in this application, words such as "for example" or "such as" are used to indicate an example, illustration, or description. Any embodiment or design solution described as "as an example" or "for example" in this application should not be construed as being preferred or advantageous over other embodiments or design solutions. To be precise, the use of the words such as "example" or "for example" is intended to present a related concept in a specific manner.

In this application, "at least one" means one or more, and "a plurality of" means two or more. "And/or" describes an association relationship between associated objects, and represents that three relationships may exist. For example, A and/or B may represent the following cases: Only A exists, both A and B exist, and only B exists, where A and B may be singular or plural. The character "/" generally indicates an "or" relationship between associated objects before and after the character. "At least one of the following" or a similar expression thereof indicates any combination of these items, including a single item or any combination of a plurality of items. For example, at least one of a, b, and c may represent a, b, c, a-b, a-c, b-c, or a-b-c, where a, b, and c may be a singular or plural number.

Currently, with development of terminal technologies, terminal devices have become a part of people's work and life. To meet the needs of users for health management, many terminal devices can provide the users with a human body data monitoring function. For example, a user may use a wearable device such as a smartwatch to measure a human body feature such as a heart rate, a heart rate variability, a respiration rate, or blood oxygen of a human body, so that the smartwatch or the like can monitor a physical condition of the user based on the human body feature.

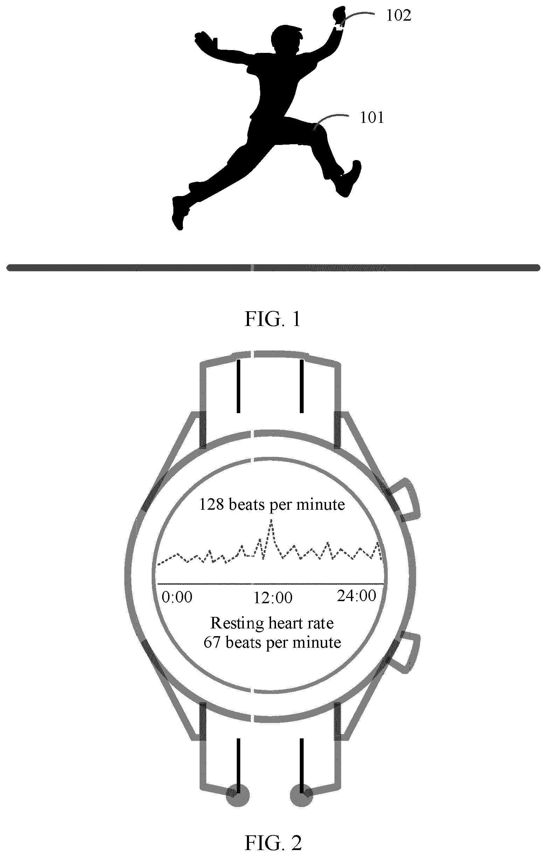

For example, FIG. 1 is a schematic diagram of a scenario according to an embodiment of this application. As shown in FIG. 1, in the moving scenario, a user 101 may measure a human body feature of the user 101 during an exercise process by using a terminal device, such as a smartwatch 102. For example, the user 101 can use the smartwatch 102 to measure a heart rate, and then the user 101 can adjust exercise intensity based on heart rate data displayed on the smartwatch 102. Alternatively, the smartwatch 102 can analyze monitored heart rate data of the user 101, and then provide the user 101 with more reasonable exercise suggestions to help the user exercise more efficiently.

The smartwatch 102 may also collect heart rate data of the user for one or more days. For example, FIG. 2 is a schematic diagram of an interface for recording heart rate based on a smartwatch according to an embodiment of this application. As shown in FIG. 2, the smartwatch 102 may record a heart rate condition of the user from 0:00 to 24:00, and can analyze to obtain data such as an exercise heart rate of the user of 128 beats/min and a resting heart rate of 67 beats/min. Further, the smartwatch 102 may provide more comprehensive health monitoring for the user based on the recorded heart rate data, or provide a data reference for the disease diagnosis of the user.

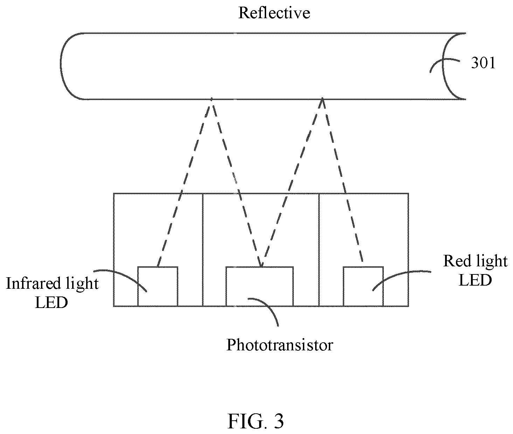

On the basis of the embodiments corresponding to FIG. 1 and FIG. 2, generally, the terminal device may monitor a human body feature based on a PPG module in the terminal device. For example, FIG. 3 is a schematic diagram of a principle of measuring human body features based on a PPG module according to an embodiment of this application. PPG may be understood as a method for irradiating light signals into skin tissue and measuring light signals reflected back through the skin tissue. As shown in FIG. 3, the PPG module 305 may include a PD such as a PD 304 and two LEDs such as an infrared light emitting diode 302 (infrared light emitting diode, IR LED) and a red light emitting diode 303 (red light emitting diode, red LED).

In the corresponding embodiment of FIG. 3, skin 301 may be irradiated with the IR LED 302 and the red LED 303 in the PPG module 305, and a light signal reflected back through the skin 301 is received with the PD 304. The PD 304 converts the light signal into an electric signal, and is subjected to analog to digital conversion (analogue to digital conversion, A/D), so as to convert the electrical signal into a digital signal that can be used by the terminal device, thereby implementing measurement of a human body feature by the terminal device.

It may be understood that a signal monitoring method base on PPG may be applied to an exercise scenario (the embodiment corresponding to FIG. 1), or may be applied to a still scenario that is continuously monitored for a long time, such as a sleep monitoring scenario.

In the moving scenario, as shown in FIG. 3, with movement of a user body, the wearable device is vibrated, causing the terminal device including the PPG module 305 and the skin 301 to form different included angles, and irregular changing of the included angle between the terminal device and the skin 301 affects the PPG signal reflected back through the skin 301, and consequently, the PD 304 in the PPG module 305 cannot receive an accurate PPG signal, and the terminal device cannot obtain valid human data based on the PPG signal.

In a sleep monitoring scenario, since operation power consumption of a PPG module in a terminal device is high, it is difficult for the terminal device to ensure long-time signal input, and thus a standby time of the terminal device may be affected. In addition, a short standby time causes the terminal device to be charged for a plurality of times, and the current terminal device has a long charging time, resulting in a short overall service time.

In view of this, embodiments of this application provide a signal monitoring structure based on PPG, and the structure include two LEDs, eight PDs and charging pins (pin). The eight PDs form a surrounding structure around outer sides of the two LEDs, and the charging pins are provided on the outer sides of the eight PDs. Each of the two LEDs is a tricolor integrated LED, and the LED can emit red light, green light, and infrared light. In this way, the terminal device can receive light signals to a maximum extent by using a surrounding structure formed by a plurality of PDs, thereby ensuring that valid human body data is obtained in a moving scenario. In addition, compared with a single PD, eight PDs can significantly increase a light receiving area, thereby reducing power consumption of the PPG module and enhancing battery life of the terminal device. In addition, the charging pins can provide a fast charging function for the terminal device, thereby increasing a service time of the terminal device. It may be understood that a quantity of PDs in the foregoing described structure is only one example. When the quantity of PDs is larger, included angles formed between adjacent PDs is smaller, and the surrounding structure formed by a plurality of PDs is closer to one circle, and light signals transmitted from the included angles between adjacent PDs are less difficult to receive, so that more light signals can be received.

It may be understood that the terminal device described above may be a wearable device, such as a smartwatch, a smart band. The terminal device may also be a smartphone, a tablet computer, or the like. Embodiments of this application impose no limitation on a specific technology and a specific device form used by the electronic device.

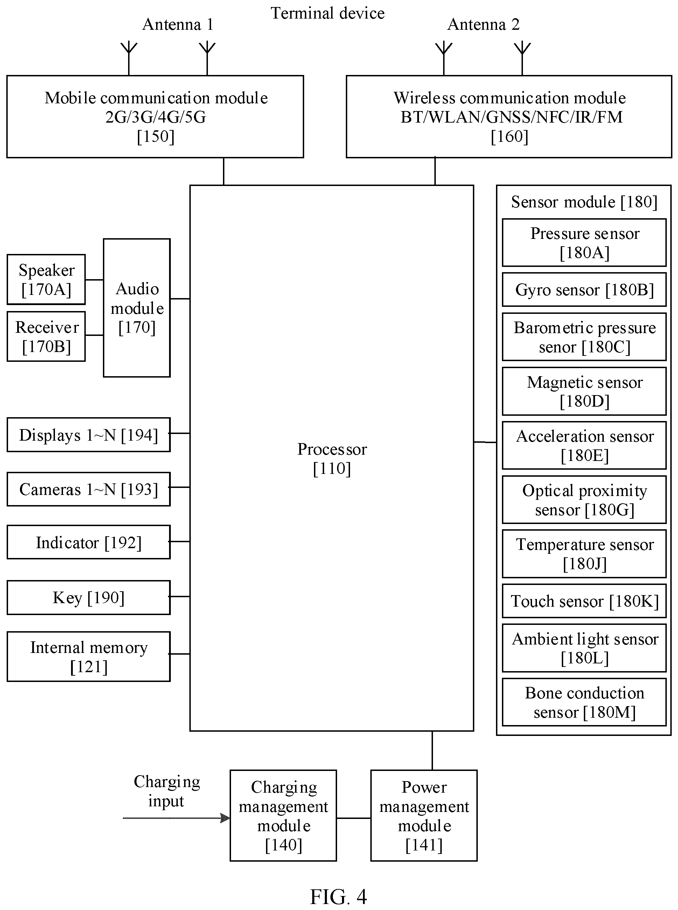

To better understand embodiments of this application, the following describes a structure of the terminal device in the embodiments of this application. For example, FIG. 4 is a schematic diagram of a structure of a terminal device according to an embodiment of this application.

The electronic device may include a processor 110, an internal memory 121, a universal serial bus (universal serial bus, USB) connector, a charge management module 140, a power management module 141, an antenna 1, an antenna 2, a mobile communication module 150, a wireless communication module 160, an audio module 170, a speaker 170A, a receiver 170B, a sensor module 180, a key 190, an indicator 192, a camera 193, a display 194, and the like. The sensor module 180 may include a pressure sensor 180A, a gyro sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, and an ambient light sensor 180L, a bone conduction sensor 180M, and the like.

It may be understood that the structure shown in this embodiment of this application does not constitute a specific limitation of the terminal device. In some other embodiments of this application, the terminal device may include more or fewer components than those shown in the figure, or combine some components, or split some components, or have different component arrangements. The components shown in the figure may be implemented by hardware, software, or a combination of software and hardware.

The processor 110 may include one or more processing units. Different processing units may be independent devices, or may be integrated into one or more processors. The processor 110 may be further provided with a memory for storing instructions and data.

The charging management module 140 is configured to receive a charging input from a charger. The charger may be a wireless charger or a wired charger. The power management module 141 is configured to be connected to the charging management module 140 and the processor 110.

A wireless communication function of the terminal device may be implemented by using the antenna 1, the antenna 2, the mobile communication module 150, the wireless communication module 160, a modem processor, a baseband processor, and the like.

The antenna 1 and the antenna 2 are configured to transmit and receive electromagnetic wave signals. The antenna in the terminal device may be configured to cover one or more communication bands. Different antennas may be multiplexed to improve antenna utilization.

The mobile communication module 150 may provide a solution to a wireless communication that is based on 2G/3G/4G/5G or the like and that is applied to the terminal device. The mobile communication module 150 may include at least one filter, a switch, a power amplifier, a low noise amplifier (low noise amplifier, LNA), and the like. The mobile communication module 150 may receive an electromagnetic wave by using the antenna 1, perform processing such as filtering and amplification on the received electromagnetic wave, and send the electromagnetic wave to the modem processor for demodulation.

The wireless communication module 160 may provide a solution to a wireless communication that is based on a wireless local area network (wireless local area networks, WLAN) (for example, wireless fidelity (wireless fidelity, Wi-Fi), bluetooth (bluetooth, BT), a global navigation satellite system (global navigation satellite system, GNSS), or frequency modulation (frequency modulation, FM)) and that is applied to the terminal device.

The terminal device implements a display function by using a GPU, the display 194, an application processor, and the like. The GPU is an image processing microprocessor and is connected to the display 194 and the application processor. The GPU is configured to perform mathematical and geometric calculations for graphics rendering.

The display 194 is configured to display an image, a video, and the like. The display 194 includes a display panel. In some embodiments, the terminal device may include one or N displays 194, where N is a positive integer greater than 1.

The terminal device may implement a photographing function by using the ISP, the camera 193, the video codec, the GPU, the display 194, the application processor, and the like.

The camera 193 is configured to capture a still image or a video. In some embodiments, the terminal device may include one or N cameras 193, where N is a positive integer greater than 1.

The internal memory 121 may be configured to store computer executable program code, and the executable program code includes an instruction. The internal memory 121 may include a storage program area and a storage data area.

The terminal device may use the audio module 170, the speaker 170A, the receiver 170B, the application processor, and the like to implement an audio function, for example, music playing and sound recording.

The audio module 170 is configured to convert digital audio information into an analog audio signal for outputting, and is further configured to convert an analog audio input into a digital audio signal. The speaker 170A, also referred to as a "horn", is configured to convert an audio electrical signal into a sound signal. The terminal device may be used to listen to music or answer a call in a hands-free mode by using the speaker 170A. The receiver 170B, also referred to as an "earpiece", is configured to convert an audio electrical signal into a sound signal. When a call is answered or audio information is listened to by using the terminal device, the receiver 170B may be put close to a human ear to listen to a voice.

The pressure sensor 180A is configured to sense a pressure signal, and may convert the pressure signal into an electrical signal. In some embodiments, the pressure sensor 180A may be disposed on the display 194. The gyro sensor 180B may be configured to determine a motion gesture of the terminal device. The barometric pressure sensor 180C is configured to measure a barometric pressure. The magnetic sensor 180D includes a Hall sensor. The acceleration sensor 180E may detect accelerations in various directions (usually on three axes) of the terminal device.

The optical proximity sensor 180G may include, for example, a light emitting diode (LED) and an optical detector PD. In embodiments of this application, the LED may be a tricolor integrated LED. The LED may be light source that emits red light, green light, infrared light, and the like. The PD may be configured to receive a light signal and process the light signal into an electrical signal. For example, in a scenario in which a heart rate is measured by using the terminal device, the PD may receive a light signal reflected back through skin tissue and process the signal into an electrical signal.

The ambient light sensor 180L is configured to sense an intensity of ambient light. The temperature sensor 180J is configured to measure temperature. The touch sensor 180K is also referred to as a "touch device". The touch sensor 180K may be disposed on the display 194. The touch sensor 180K and the display 194 form a touchscreen, which is also referred to as a "touch screen". The bone conduction sensor 180M may collect a vibration signal.

The key 190 includes a power key, a volume key, and the like. The key 190 may be a mechanical key, or may be a touch key. The terminal device may receive a key input and generate a key signal input related to user settings and function control of the terminal device. The indicator 192 may be an indicator light, which may be configured to indicate a charging state and a power change, or may be configured to indicate a message, a missed call, a notification, or the like.

The following describes, by using specific embodiments, in detail the technical solutions of this application and how the technical solutions of this application resolve the foregoing technical problems. The following several specific embodiments may be implemented independently, or may be combined with each other. For same or similar concepts or processes, details may not be described in some embodiments again.

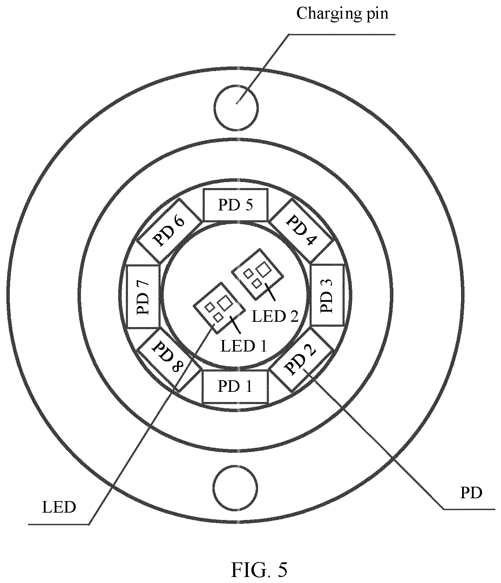

For example, FIG. 5 is a schematic diagram of a structure of a smartwatch based on two LEDs+eight PDs+wired fast charging according to an embodiment of this application. In the embodiment corresponding to FIG. 5, exemplary descriptions are made by using an example in which a terminal device is a smartwatch, and this example does not constitute a limitation of embodiments of this application. It may be understood that the following description of the structure of the terminal device is described by using a smartwatch as an example. Details are not described below. The wired fast charging may be replaced by wireless charging, wireless fast charging, or other charging forms. This is not limited in this embodiment of this application.

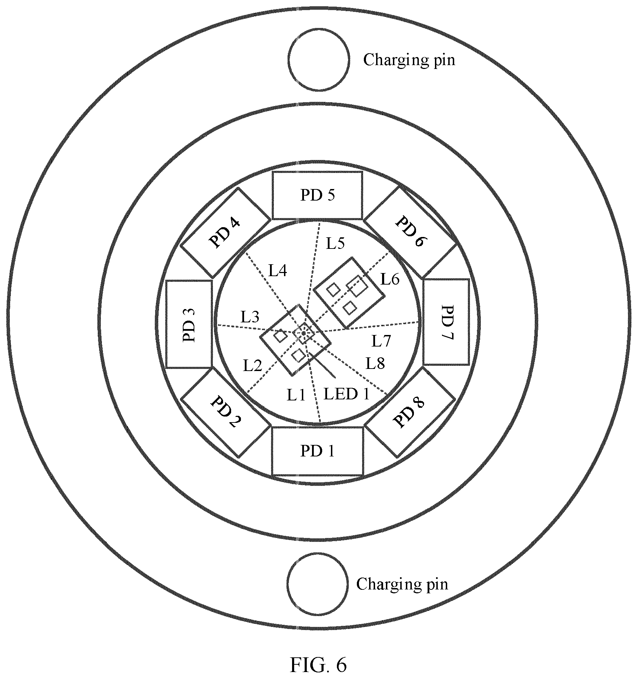

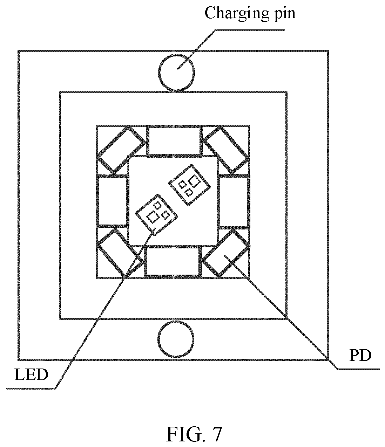

For example, the smartwatch may have a circular structure (embodiments corresponding to FIG. 5 and FIG. 6) or a square structure (an embodiment corresponding to FIG. 7).

As shown in FIG. 5, an example in which the smartwatch is of a circular structure and a PPG module with a circular structure is disposed on a back of the smartwatch is used for description. The PPG module of the circular structure may include two tricolor integrated LEDs, eight PDs, and two charging pins. The two tricolor integrated LEDs are provided in the innermost part of the PPG module, and the two tricolor integrated LEDs can both be configured to emit light signals, such as red light, green light, and infrared light. As shown in FIG. 5, the two tricolor integrated LEDs are provided with eight PDs arranged in a concentric circle with a surrounding structure, and the eight PDs satisfy an eight-equal distribution with a midpoint of a connecting line of the two tricolor integrated LEDs as a circle center, and each PD is used to receive a light signal reflected back through skin tissue. Outermost sides of the eight PDs are provided with two charging pins for wired fast charging. The two charging pins may be a positive electrode and a negative electrode respectively.

As shown in FIG. 5, the two tricolor integrated LEDs may include: an LED 1 and an LED 2. The eight PDs in the surrounding structure may include a PD 1, a PD 2, a PD 3, a PD 4, a PD 5, a PD 6, a PD 7, and a PD 8. It may be understood that the surrounding structure formed by the eight PDs may occupy angles of a circle to a maximum extent, and the eight PDs may be tightly arranged around to implement the surrounding structure, so that a light signal transmitted from any direction may be received as much as possible by at least one PD of the eight PDs. It may be understood that if a quantity of PDs is sufficient, an included angle between adjacent PDs is almost zero, and the surrounding structure formed by the plurality of PDs may occupy almost all of angles of a circle, so that almost no light signal that is transmitted through the included angle between adjacent PDs is difficult to receive by the PD, thereby ensuring that the PD can receive a light signal transmitted in any direction.

It may be understood that the tricolor integrated LED can be realized by combining three LEDs into one, thereby saving an area occupied by the LEDs in the PPG module. In addition, in some scenarios in which a plurality of light signals are required, consistency of light paths of the plurality of light signals can be ensured. For example, in a scenario in which blood oxygen is measured by using a smartwatch including a tricolor integrated LED, a red LED and an IR LED in the tricolor integrated LED may be used to alternately emit red light and infrared light. Since the red LED and the IR LED are arranged closely in the tricolor integrated LED, consistency of light paths of the two light signals can be ensured as much as possible, and further calculation of the two light signals on the light paths of the consistency can be performed by the terminal device to obtain more accurate human data.

Based on this, the terminal device can use a surrounding structure formed by eight PDs to receive light signals to a maximum extent, thereby ensuring that valid human body data can be obtained in a moving scenario.

Optionally, compared with a single PD, the PPG structure using eight PDs can significantly increase a light receiving area, thereby reducing power consumption of the PPG module and enhancing battery life of the terminal device. In addition, the charging pins can provide a fast charging function for the terminal device, thereby increasing a service time of the terminal device.

An operating principle of FIG. 5 is described below. On the basis of the embodiment corresponding to FIG. 5, the two LEDs and the eight PDs in the PPG structure may constitute different light paths over distances from the LEDs to the PDs. For example, light signals from a light outlet in each LED to the PDs may constitute different light paths. For example, FIG. 6 is a schematic diagram of a structure of another smartwatch based on two LEDs+eight PDs+wired fast charging according to an embodiment of this application.

As shown in FIG. 6, if a light outlet in the LED 1 emits a light signal, the light signal reflected back through the skin tissue may be received by the PD 1, the PD 2, the PD 3, the PD 4, the PD 5, the PD 6, the PD 7, and the PD 8; and signal transmission between the LEDs (for example, the LED 1) and each PD (for example, the PD 1 to the PD 8) may constitute eight light paths. Specifically, when a light signal is transmitted from a light outlet in the LED 1 and the PD 2 receives the light signal, a light path from the light outlet in the LED 1 to the PD 2, for example, L2, may be understood as a near light path. Similarly, a light path from the light outlet in the LED 1 to the PD 3, for example, L3; a distance from the light outlet in the LED 1 to the PD 4, for example, L4; a distance from the light outlet in the LED 1 to the PD 1, for example, L1; and a distance from the light outlet in the LED 1 to the PD 8, for example, L8, each may be understood as a medium-distance light path. A light path from the light outlet in the LED 1 to the PD 5, for example, L5; a distance from the light outlet in the LED 1 to the PD 6, for example, L6; a distance from the light outlet in the LED 1 to the PD 7, for example, L7, each may be understood as a remote light path. In structures of terminal devices of different sizes, a length of the near light path, a length of the medium-distance light path, and a length of the remote light path may be different. For example, in a smartwatch structure, a light path of less than 3.5 mm may be referred to as a near light path, a light path of greater than 4.5 mm may be referred to as a remote light path, and a light path of greater than or equal to 3.5 mm and less than or equal to 4.5 mm may be referred to as a medium-distance light path.

It may be understood that the plurality of light paths may provide different signal intensity for different application scenarios. For example, generally, when blood oxygen is monitored by using a PPG module in a smartwatch, light signals on a medium-distance light path and a remote light path may be selected as input signals for monitoring the blood oxygen. Alternatively, when a heart rate is monitored by using the PPG module in the smartwatch, light signals on the medium-distance light path and a near light path can be selected as input signals for monitoring the heart rate. This avoids calculating intensity of signals received in all light paths, and the intensity of the signals in the light paths corresponding to the scenario can be obtained, thereby saving a calculation overhead of the terminal device.

It may be understood that the example of the near light path, the medium-distance light path, and the remote light path in FIG. 6 and FIG. 6 that are provided in this embodiment of this application is merely used as reference, and cannot be used as a limitation of this embodiment of this application.

Based on this, the PPG module is formed by two LEDs and eight PDs, so that the eight PDs can receive light signals in a plurality of light paths, increase a total light receiving area of the PPG structure, and reduce luminous brightness of the LEDs required for generating a same photocurrent, thereby reducing power consumption of the PPG module, and prolonging battery life of the terminal device.

On the basis of the corresponding embodiment of FIG. 5, in a possible implementation, the smartwatch may alternatively have a square structure. For example, FIG. 7 is a schematic diagram of a structure of yet another smartwatch based on two LEDs+eight PDs+wired fast charging according to an embodiment of this application.

As shown in FIG. 7, a back of the smartwatch may be provided with a PPG module having a square structure. The PPG module of the square structure may include two tricolor integrated LEDs, eight PDs, and two charging pins. Specifically, the two tricolor integrated LEDs are provided in an innermost part of the PPG module, and the two tricolor integrated LEDs can both be used to emit light signals, such as red light, green light, and infrared light. As shown in FIG. 7, outer sides of the two tricolor integrated LEDs are provided with eight PDs arranged in a concentric square with a surrounding structure, and outermost sides of the eight PDs are provided with two charging pins for wired fast charging. It may be understood that a shape of the terminal device and a shape of the PPG module may include other content based on an actual scenario. This is not limited in this embodiment of this application. For example, when the terminal device is of a circular structure, the PPG module in the terminal device may be of a circular structure or a square structure; or when the terminal device is of a square structure, the PPG module in the terminal device may be of a square or circular structure.

It may be understood that a quantity of PDs and LEDs in the PPG structure is not limited in this embodiment of this application. For example, the quantity of PDs may be more than 8. For example, the quantity of PDs may be 9, 10, 12, or 16, or the quantity of LEDs may be 3 or 4, or the LEDs may be four-color integrated LEDs or multi-color integrated LEDs.

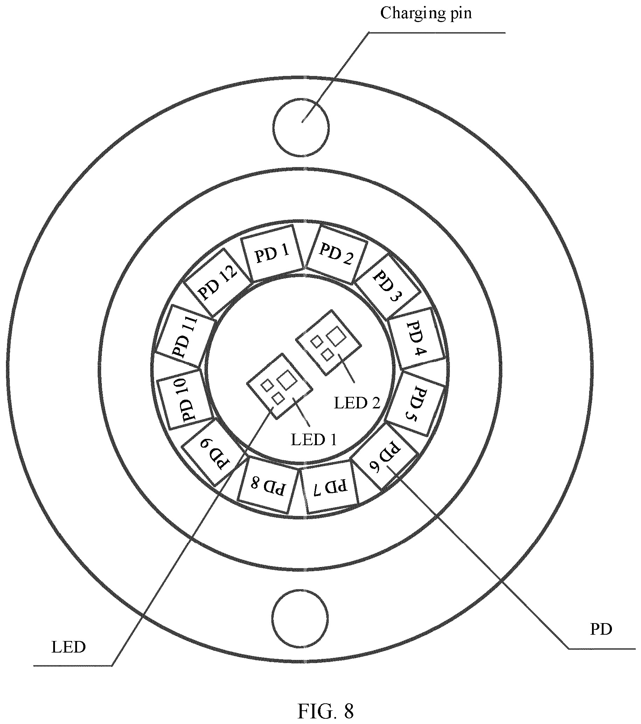

For example, FIG. 8 is a schematic diagram of a structure of a smartwatch based on two LEDs+12 PDs+wired fast charging according to an embodiment of this application. As shown in FIG. 8, a back of the smartwatch is provided with a PPG module having a circular structure. The PPG module may include two tricolor integrated LEDs, 12 PDs, and two charging pins. Outer sides of the two tricolor integrated LEDs are provided with 12 PDs arranged in a concentric circle with a surrounding structure, and the 12 PDs satisfy a twelve-equal distribution with a midpoint of a connecting line of the two tricolor integrated LEDs as a circle center. It may be understood that an internal structure and a function of the tricolor integrated LEDs and the two charging pins in FIG. 8 are the same as an internal structure and a function of the tricolor integrated LEDs and the two charging pins in FIG. 5 (or FIG. 6 or FIG. 7), and details are not described herein again.

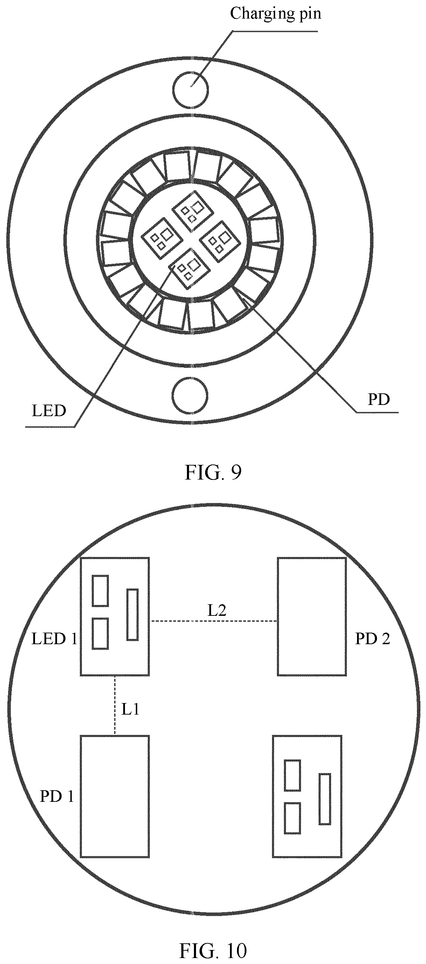

For example, FIG. 9 is a schematic diagram of a structure of a smartwatch based on four LEDs+16 PDs+wired fast charging according to an embodiment of this application. As shown in FIG. 8, a back of the smartwatch is provided with a PPG module having a circular structure. The PPG module may include four tricolor integrated LEDs, 16 PDs, and two charging pins. The four tricolor integrated LEDs are provided with 16 PDs arranged in a concentric circle with a surrounding structure, and the 16 PDs satisfy a sixteen-equal distribution with a midpoint of a connecting line of any two tricolor integrated LEDs among the four tricolor integrated LEDs as a circle center. It may be understood that an internal structure and a function of the tricolor integrated LEDs and the two charging pins in FIG. 9 are the same as an internal structure and a function of the tricolor integrated LEDs and the two charging pins in FIG. 5 (or FIG. 6 or FIG. 7), and details are not described herein again.

It may be understood that, as shown in FIG. 5 to FIG. 9, in the PPG structure of the terminal device, it needs to ensure that a plurality of PDs constitute an enclosed structure.

Based on this, the terminal device can receive light signals to a maximum extent by using the surrounding structure formed by the plurality of PDs, and it is ensured that valid human body data can be obtained in a moving scenario. In addition, compared with a single PD, eight PDs can significantly increase a light receiving area, thereby reducing power consumption of the PPG module and enhancing battery life of the terminal device. In addition, the charging pins can provide a fast charging function for the terminal device, thereby increasing a service time of the terminal device.

In a possible implementation, a PPG structure including two PDs and two LEDs is provided. APPG module may include two PDs and two LEDs. For example, FIG. 10 is a schematic diagram of a structure of a smartwatch based on two LEDs+two PDs according to an embodiment of this application.

As shown in FIG. 10, a PPG module may include two tricolor integrated LEDs and two PDs. The two tricolor integrated LEDs and the two PD are distributed on the circle at intervals. The tricolor integrated LED may emit red light, green light, and infrared light.

For example, if an LED 1 emits a light signal, a light signal reflected back through skin tissue may be received by a PD 1 and a PD 2, and two paths may be formed between the LED1 and each PD. A light signal is emitted by the LED 1, and a light signal is received by the PD 1, and a light path, for example, L1, between the LED 1 and the PD 2 may be understood as a near light path. A light signal is emitted by the LED 1, and a light signal is received by the PD 2, and a light path, for example, L2, between the LED 1 and the PD 2 may be understood as a remote light path.

In summary, comparison between technical indicators of the PPG structure of the two LEDs+eight PDs+charging pins (the embodiment corresponding to FIG. 5) and the PPG structure of the two LEDs+two PDs (the embodiment corresponding to FIG. 10) may be shown in the following Table 1:

**Table 1 Comparison between technical indicators of two LEDs+eight PDs+charging pins and two LEDs+two PDs**

| | Path quantity | Available light paths | Power consumption |
|---|---|---|---|
| Two LEDs+eight PDs+charging pins | 8 | Near light path, medium-distance light path, and remote light path | <0.5X |
| Two LEDs+two PDs | 2 | Near light path and remote light path | X |

Based on this, when a PPG structure including eight PDs is provided in a terminal device, compared with a PPG structure including two PDs, the PPG structure including the eight PDs can increase a light receiving area and reduce luminous brightness of the LEDs required for generating a same photocurrent. In addition, since power consumption of the LEDs occupies most of power consumption of the PPG module, the PPG structure including the eight PDs can significantly reduce the power consumption of the PPG module, thereby prolonging battery life of the terminal device.

Based on the embodiment corresponding to FIG. 5, in a possible implementation, the control method provided in this embodiment of this application may be applied to the foregoing signal monitoring structure based on PPG.

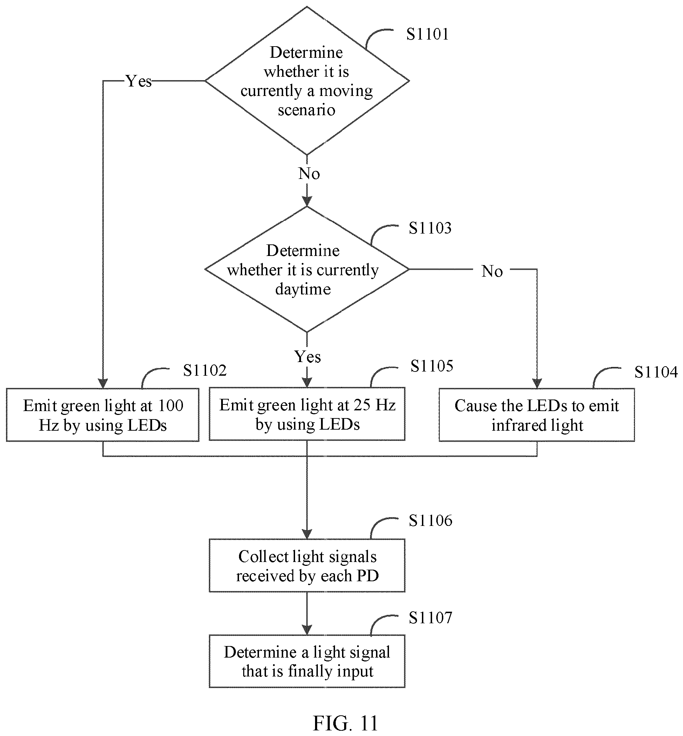

For example, FIG. 11 is a schematic flowchart of a control method according to an embodiment of this application. In the embodiment corresponding to FIG. 11, an example in which a heart rate is monitored by using a PPG structure in a terminal device is used for description. This example does not constitute a limitation of this embodiment of this application.

As shown in FIG. 11, the method for monitoring the heart rate by the terminal device may include the following steps.

S1101: The terminal device determines whether the terminal device is currently in a moving scenario.

In this embodiment of this application, when the terminal device determines that the terminal device is currently in a moving scenario, the terminal device may perform a step shown in S1102. When the terminal device determines that the terminal device is not currently in a moving scenario, or it is understood that the terminal device is currently in a still scenario, the terminal device may perform a step shown in S1103.

For example, the terminal device may determine whether the current terminal device is in a moving scenario by using a motion sensor of the terminal device, such as an acceleration sensor, or a setting of a user for the current scenario. For example, when the terminal device receives an operation of setting a riding mode by a user, the terminal device may determine that the terminal device is currently in a moving scenario.

S1102: The terminal device causes the LED to emit green light at 100 Hz.

For example, when it is detected that the terminal device is in a moving state, the terminal device may control intensity of a light signal emitted by the LED. In this embodiment of this application, intensity of the light signal may be determined by a frequency of light emitted by the LED, and a frequency of the emitted light signal may be different depending on an application scenario.

The terminal device may emit green light by using the LED 1 and/or the LED 2 as shown in FIG. 5. Intensity of the light signals received by a PD in the PPG module can be improved by using the LED 1 and the LED 2 to emit light simultaneously. S1103: The terminal device determines a current lighting environment.

In this embodiment of this application, when the terminal device determines that current lighting is weak, the terminal device may perform a step shown in S1104. When the terminal device determines that current lighting environment is strong, the terminal device may perform a step shown in S1105.

For example, the terminal device may determine a lighting environment of the current terminal device by using an ambient light sensor of the terminal device, such as detecting a lighting level of a current environment; or the terminal device may read current time information and determine the current lighting environment based on the time information, for example, when the terminal device reads that the current time is 2: 00 a.m. (ante meridiem, AM), the terminal device can determine that current lighting is weak.

It may be understood that, that the terminal device determines the current lighting environment may be used to select an appropriate LED light emission source based on different lighting scenarios. For example, a heart rate may be monitored by using an LED to emit red or green during a day when lighting is strong; or a heart rate may be monitored by using an LED to emit infrared light at night when lighting is weak.

S1104: The terminal device causes the LED to emit infrared light.

For example, the terminal device may emit infrared light using the LED 1 and/or the LED 2 shown in FIG. 5.

It may be understood that when lighting is strong (or understood as that light intensity is greater than or equal to a light intensity threshold), for example, in the daytime, the terminal device may use visible light, such as green light or red light, to monitor the heart rate, and the visible light may be perceived by the user; and when lighting is weak (or understood as that light intensity is less than the light intensity threshold), for example, at night, the terminal device may monitor the heart rate by using invisible light such as infrared light, to prevent the user from being affected by the visible light.

S1105: The terminal device causes the LED to emit green light at 25 Hz.

For example, the terminal device may emit green light using the LED 1 and/or the LED 2 shown in FIG. 5.

S1106: The terminal device collects light signals received by each PD.

S1107: The terminal device determines a light signal that is finally input.

For example, a method in which a terminal device obtains a light signal finally used for human body feature monitoring based on the light signals received by each PD may include the following three types.

Method 1: The terminal device can obtain a light signal with the strongest signal intensity among the light signals received by each PD as a light signal that is finally input.

In this way, the terminal device can screen out weaker light signals and achieve accurate measurement of human body features based on the strongest light signal.

Method 2: The terminal device can calculate an average signal of the light signals received by each PD, and use the average signal as a light signal that is finally input.

For example, the terminal device removes a light signal with the strongest signal intensity and a light signal with weakest signal intensity from the obtained light signals received by the plurality of PDs, calculates an average value of the remaining one or more light signals, and uses the average signal as a light signal that is finally input. For example, when selecting light signals received from a medium-distance light path and a remote light path as input signals for monitoring blood oxygen, all light signals received from the medium-distance light path and the remote light path can be obtained, a light signal with the strongest intensity and a light signal with weakest signal intensity are removed, and an average value of remaining light signals is taken as an input signal for detecting blood oxygen.

This avoids a case in which a signal received by the PD is inaccurate due to PD damage or the like. Based on an average light signal, a more accurate monitoring result of a human body feature can be obtained, thereby saving a calculation overhead when the terminal device calculates an input signal.

Method 3: The terminal device may set a weight for each PD, and obtain a light signal of a PD with a highest weight as a light signal that is finally input.

For example, the terminal device may record light signals as final inputs as well as PDs corresponding to the light signals in a plurality of times of monitoring. The terminal device may set a relatively high weight for a PD corresponding to the light signals that are used as the final inputs for a large quantity of times based on the historical monitoring record, and set a relatively low weight for a PD of the light signals that are used as the final inputs for a small quantity of times. Further, the terminal device may use a light signal received by a PD with a highest weight as the light signal that is finally input. Alternatively, the terminal device may use a weighted average value of a weight corresponding to each PD and a light signal of each PD as a light signal that is finally input.

The terminal device may record which PD of the plurality of PDs usually obtains a signal that is finally input when different scenarios are recorded or when different human body features are measured. For example, when a terminal device measures a heart rate in a moving scenario, a light signal received by a PD 3 is usually obtained as an input signal for heart rate measurement, and a higher weight can be set for the PD 3. Alternatively, in this scenario, the terminal device may also set a lower weight for a PD of the light signals that are used as the final inputs for a small quantity of times.

In an implementation, when a heart rate is measured in the current moving scenario, the terminal device may obtain a light signal received by the PD 3 with a higher weight as a green light signal that is finally input during heart rate measurement.

In another implementation, when a heart rate is measured in the current moving scenario, the terminal device may obtain the light signals of the plurality of PDs, calculate a weighted average value of the light signals and the weights of the plurality of PDs based on the weights of the plurality of PDs, and further as a green light signal finally input when the heart rate is measured based on a result of the weighted average value.

It may be understood that in other scenarios, the method for measuring a human body feature based on weights of PDs may include other content. This is not limited in this embodiment of this application.

In this way, the terminal device can set a weight for the PD based on a history monitoring record, and obtain a more accurate monitoring result of the human body feature based on weights of PDs, thereby avoiding a plurality of times of calculations of input signals by the terminal device, and saving a calculation overhead. It may be understood that a light-emitting strategy provided in this embodiment of this application may include other content based on an actual scenario. This is not limited in this embodiment of this application.

In a possible implementation, a wearable device based on PPG provided in an embodiment of this application may also be used to monitor blood oxygen. For example, the LED 1 (or the LED 2) shown in FIG. 5 may be used to alternately emit red light and infrared light, and PDs are used to receive the red light signal and the infrared light signal reflected back through skin tissue, so that the terminal device may monitor blood oxygen based on two types of light signals.

In a possible implementation, a respiration rate may also be monitored by using the wearable device based on PPG provided in this embodiment of this application. For example, green light may be emitted by using the LED 1 (and/or the LED 2) shown in FIG. 5, and a green light signal reflected back through skin tissue is received with each PD, so that the terminal device may monitor the respiration rate based on the green light signal.

Based on this, in different scenarios, the terminal device may obtain a valid PPG signal based on the PPG structure of the annular surrounding structure of the plurality of PDs.

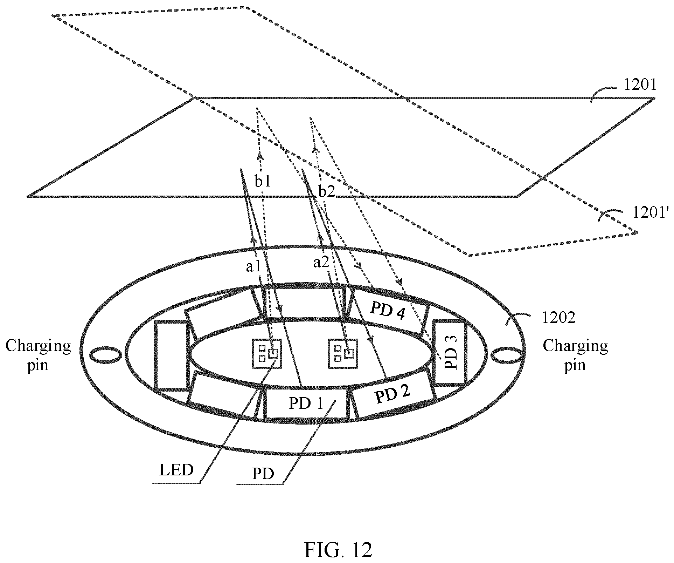

For example, FIG. 12 is a schematic diagram of receiving a light signal through PPG in a moving state according to an embodiment of this application. In the embodiment corresponding to FIG. 12, a process in which a PPG module receives a signal in a moving state and measures a heart rate based on a signal is described by using an example in which green light in a smartwatch is used to measure a heart rate. This example does not constitute a limitation of this embodiment of this application.

As shown in FIG. 12, the scenario may include a PPG module 1202 in a smartwatch and skin 1201. A structure of the PPG module may be two LEDs+eight PDs+wired fast charging, and the structure of the PPG module is not described herein again.

In a still state of a user, both LEDs in the smartwatch may emit green light signals such as a1 and a2, which reach the skin 1201 and are reflected to the PPG module, the reflected a1 may be received by a PD 1, and the reflected a2 may be received by a PD 2, so that the smartwatch may calculate a heart rate of the user based on the green light signals received by the PD 1 and the PD 2.

Further, as shown in FIG. 12, when a user is wearing the smartwatch to run, ride, or do other exercise, the skin may not fit closely with the smartwatch due to movement of the user body, for example, the skin 1201 may be deflected at an angle, for example, to skin 1201'. The two LEDs in the smartwatch continue to emit green light signals b1 and b2 to the skin 1201', at which an angle deflection occurs, and an angle of a PPG signal reflected back through the skin deviates due to shaking of the skin. In this case, the smartwatch can obtain, at all angles, PPG signals received through light paths of PDs to a maximum extent by using an annular surrounding structure of the plurality of PDs in the PPG module. For example, a PD 4 may be used to receive b1 reflected back through the skin 1201', and the PD3 may be used to receive the b2 reflected back through the skin 1201', so that the smartwatch can calculate an accurate heart rate based on the obtained green light signals. In addition, a greater quantity of PDs in the PPG module indicates that a measured human body feature is closer to a true value.

For example, when a user wears the wearable device based on PPG according to this embodiment of this application while sleeping, for example, the smartwatch, to measure human body data, since the PPG module in the smartwatch includes a plurality of PDs, a signal receiving area can be significantly increased compared with that of a single PD, thereby reducing power consumption of the PPG module, and ensuring battery life of the smartwatch. In addition, the smartwatch according to this embodiment of this application can further support a fast charging function of the device, thereby ensuring a long battery life of the smartwatch, and improving a service time of the smartwatch.

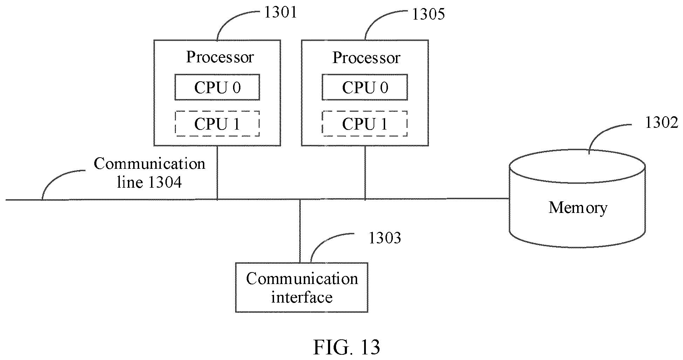

For example, FIG. 13 is a schematic diagram of a hardware structure of a control device according to an embodiment of this application. As shown in FIG. 13, the control device includes a processor 1301, a communication line 1304, and at least one communication interface (for example, in FIG. 13, a communication interface 1303 is used as an example for description).

The processor 1301 may be a general-purpose central processing unit (central processing unit, CPU), a microprocessor, an application-specific integrated circuit (application-specific integrated circuit, ASIC), or one or more integrated circuits for controlling program execution in the solutions of this application.

The communication line 1304 may include a circuit for transmitting information between the foregoing components.

The communication interface 1303 uses any apparatus such as a transceiver to communicate with another device or a communication network, such as an Ethernet or a wireless local area network (wireless local area networks, WLAN).

Possibly, the control device may further include a memory 1302.

The memory 1302 may be a read-only memory (read-only memory, ROM) or another type of static storage device capable of storing static information and instructions, a random access memory (random access memory, RAM) or another type of dynamic storage device capable of storing information and instructions, or an electrically erasable programmable read-only memory (electrically erasable programmable read-only memory, EEPROM), a compact disc read-only memory (compact disc read-only memory, CD-ROM) or another optical disc memory, a compact disc memory (including a compact disc, a laser disc, an optical disc, a digital versatile discs, a Blu-ray disc, and the like), magnetic disc storage medium or another magnetic storage device, or any other medium that can be used to carry or store desired program code in the form of instructions or data structures and that can be accessed by a computer, but is not limited thereto. The memory may be stand-alone and connected to the processor through the communication line 1304. The memory may alternatively be integrated with the processor.

The memory 1302 is configured to store a computer executable instruction for performing the solution in this application, and the processor 1301 controls execution. The processor 1301 is configured to execute computer-executable instructions stored in the memory 1302 to implement the control method according to the embodiments of this application.

Possibly, the computer-executable instructions in this embodiment of this application may alternatively be referred to as application code, which is not specifically limited in this embodiment of this application.

During specific implementation, in an embodiment, the processor 1301 may include one or more CPUs, such as a CPU 0 and a CPU 1 in FIG. 13.

During specific implementation, in an embodiment, the control device may include a plurality of processors, such as a processor 1301 and a processor 1305 in FIG. 13. Each of these processors may be a single-core (single-CPU) processor or a multi-core (multi-CPU) processor. The processor herein may refer to one or more third devices, circuits, and/or processing cores for processing data (such as computer program instructions).

In the foregoing embodiments, the instructions stored in the memory for execution by the processor may be implemented in the form of a computer program product. The computer program product may be written in the memory in advance, or may be downloaded and installed in the memory in the form of software.

The computer program product includes one or more computer instructions. When the computer program instructions are loaded and executed on the computer, the procedure or functions according to embodiments of this application are completely or partially generated. The computer may be a general-purpose computer, a special-purpose computer, a computer network, or another programmable apparatus. The computer instructions may be stored in a computer-readable storage medium or transmitted from one computer readable storage medium to another computer-readable storage medium. For example, the computer instructions may be transmitted from one network site, computer, server or data center to another network site, computer, server or data center in a wired (such as coaxial cable, optical fiber, or digital subscriber line (DSL)) or wireless (such as infrared, wireless, or microwave) manner. The computer-readable storage medium may be any available medium accessible by a computer, or a data storage device such as a server or a data center, integrating one or more available media. For example, an available medium may include a magnetic medium (for example, a floppy disk, a hard disk, or a magnetic tape), an optical medium (for example, a digital versatile disc (digital versatile disc, DVD)), or a semiconductor medium (for example, a solid state disk (solid state disk, SSD)).

An embodiment of this application further provides a computer-readable storage medium. All or some of the methods described in embodiments may be implemented by software, hardware, firmware, or any combination thereof. The computer-readable medium may include a computer storage medium and a communication medium, and may alternatively include any medium that may transmit a computer program from one place to another. The storage medium may be any target medium accessible by the computer.

In a possible design, the computer-readable medium may include a compact disc read-only memory (compact disc read-only memory, CD-ROM), a RAM, a ROM, an EEPROM, or another optical disc memory; and the computer-readable medium may include a magnetic disc memory or another disk storage device. In addition, any connecting line may be appropriately referred to as a computer-readable medium. For example, if software is transmitted from a website, a server or another remote source by using a coaxial cable, an optical fiber cable, a twisted pair, a DSL or wireless technologies (for example, infrared, radio, and microwave), the coaxial cable, the optical fiber cable, the twisted pair, the DSL or wireless technologies such as infrared, radio and microwave are included in the definition of medium. As used herein, magnetic and optical discs include a compact disc (CD), a laser disc, an optical disc, a digital versatile disc (digital versatile disc, DVD), a floppy disc, and a Blu-ray disc, and the magnetic disc usually reproduces data magnetically, while the optical disc reproduces data optically using lasers.

The foregoing combinations should also be included in the scope of the computer-readable medium. The foregoing descriptions are merely specific implementations of this invention. However, the protection scope of the present invention is defined by the scope of the claims.

## Claims

1. A wearable device based on photoplethysmography, PPG, wherein the wearable device comprises a PPG module (305) and a processor (110); and the PPG module (305) comprises a plurality of light emitting diodes, LEDs and a plurality of photodiodes, PDs;
the plurality of PDs are distributed around the plurality of LEDs in a surrounding structure; each of the LEDs is configured to emit light signals; and the light signals comprise: a green light signal, a red light signal, and/or an infrared light signal;
each PD is configured to: receive the light signals, and transmit the light signals to the processor (110); and
the processor (110) is configured to obtain a heart rate feature, a blood oxygen feature, and/or a respiration rate feature based on the light signals received from the plurality of PDs;
**characterized in that** the LED is a tricolor integrated LED in which red light, green light and infrared light are combined
and **in that** the processor (110) is further configured to:
control the LED to emit a green light signal and/or a red light signal when it is detected that an ambient light intensity is greater than or equal to a light intensity threshold;
control the LED to emit an infrared light signal when it is detected that the ambient light intensity is less than the light intensity threshold; and
control frequency of the light signal emitted by the LED when it is detected that the wearable device is in a moving state.

2. The wearable device according to claim 1, wherein the processor (110) is further configured to: obtain a third light signal having the strongest signal intensity from the light signals from the plurality of PDs, and obtain the heart rate feature, the blood oxygen feature, and/or the respiration rate feature from the third light signal; or
the processor (110) is further configured to: obtain a fourth light signal by calculating an average value of the light signals from the plurality of PDs, and obtain the heart rate feature, the blood oxygen feature, and/or the respiration rate feature from the fourth light signal; or
the processor (110) is further configured to: obtain a fifth light signal by weighting calculation based on the light signals from the plurality of PDs and a weight of each PD, and obtain the heart rate feature, the blood oxygen feature, and/or the respiration rate feature based on the fifth light signal; wherein the weight of each PD is determined by the processor (110) based on a quantity of times that the light signals emitted by each PD are used as an obtaining basis in a history record, or the weight of each PD is preset.

3. The wearable device according to claim 1, wherein the PPG module (305) further comprises fast charging pins; the fast charging pins are provided outside the plurality of PDs; and the fast charging pins are configured to provide a fast charging interface for the wearable device.

4. The wearable device according to claim 1, wherein a quantity of the LEDs is two; a quantity of PDs is eight; and the eight PDs are arranged in an eight-equal distribution with a midpoint of a connecting line of the two LEDs as a circle center.

5. The wearable device according to claim 1, wherein the structure of the PPG module (305) is a concentric circular structure or a concentric square structure.

6. A control method, wherein the method is applied to the wearable device according to any one of claims 1 to 5, and the method comprises:
receiving light signals from a plurality of PDs; and
obtaining a heart rate feature, a blood oxygen feature, and/or a respiration rate feature based on the light signals received from the plurality of PDs, wherein each PD is configured to: receive light signals transmitted by an LED, and transmit the light signals to a processor (110); and the light signals comprise a green light signal, a red light signal, and/or an infrared light signal;
wherein the method further comprises:
controlling the LED to emit a green light signal and/or a red light signal when it is detected that an ambient light intensity is greater than or equal to a light intensity threshold;
controlling the LED to emit an infrared light signal when it is detected that the ambient light intensity is less than the light intensity threshold; and
controlling frequency of the light signal emitted by the LED when it is detected that the wearable device is in a moving state.

7. The method according to claim 6, wherein the obtaining a heart rate feature, a blood oxygen feature, and/or a respiration rate feature based on the light signals received from the plurality of PDs comprises:
obtaining a third light signal having the strongest signal intensity from the light signals from the plurality of PDs, and obtaining the heart rate feature, the blood oxygen feature, and/or the respiration rate feature from the third light signal; or
obtaining a fourth light signal by calculating an average value of the light signals from the plurality of PDs, and obtaining the heart rate feature, the blood oxygen feature, and/or the respiration rate feature from the fourth light signal; or
obtaining a fifth light signal by weighting calculation based on the light signals from the plurality of PDs and a weight of each PD, and obtaining the heart rate feature, the blood oxygen feature, and/or the respiration rate feature based on the fifth light signal; wherein the weight of each PD is determined by the processor (110) based on a quantity of times that the light signals emitted by each PD are used as an obtaining basis in a history record, or the weight of each PD is preset.

## Patentansprüche

1. Ein tragbares Gerät basierend auf der Photoplethysmographie (PPG), wobei das tragbare Gerät ein PPG-Modul (305) und einen Prozessor (110) umfasst; und das PPG-Modul (305) eine Vielzahl von Leuchtdioden (LEDs) und eine Vielzahl von Photodioden (PDs) umfasst;
die Vielzahl von PDs ist um die Vielzahl von LEDs in einer umgebenden Struktur verteilt;
jede der LEDs ist dazu ausgelegt, Lichtsignale abzugeben; und die Lichtsignale umfassen: ein grünes Lichtsignal, ein rotes Lichtsignal und/oder ein Infrarot-Lichtsignal;
jede PD ist so konfiguriert, dass sie: die Lichtsignale empfängt und die Lichtsignale an den Prozessor (110) weiterleitet; und
der Prozessor (110) ist dazu ausgelegt, basierend auf den von der Vielzahl von PDs empfangenen Lichtsignalen eine Herzfrequenzeigenschaft, eine Blutsauerstoffeigenschaft und/oder eine Atemfrequenzeigenschaft zu ermitteln;
**dadurch gekennzeichnet, dass** die LED eine dreifarbige integrierte LED ist, bei der rotes Licht, grünes Licht und Infrarotlicht kombiniert sind, und dass der Prozessor (110) ferner dazu ausgelegt ist:
die LED dazu zu steuern, ein grünes Lichtsignal und/oder ein rotes Lichtsignal abzugeben, wenn erkannt wird, dass die Umgebungslichtintensität größer oder gleich einem Lichtintensitätsschwellenwert ist;
die LED dazu zu steuern, ein Infrarot-Lichtsignal abzugeben, wenn erkannt wird, dass die Umgebungslichtintensität kleiner als der Lichtintensitätsschwellenwert ist; und
die Frequenz des von der LED abgegebenen Lichtsignals zu steuern, wenn erkannt wird, dass sich das tragbare Gerät im Bewegungszustand befindet.

2. Das tragbare Gerät nach Anspruch 1, wobei der Prozessor (110) ferner dazu ausgelegt ist: ein drittes Lichtsignal mit der stärksten Signalintensität aus den Lichtsignalen der Vielzahl von PDs zu ermitteln und die Herzfrequenzeigenschaft, die Blutsauerstoffeigenschaft und/oder die Atemfrequenzeigenschaft aus dem dritten Lichtsignal zu ermitteln; oder
der Prozessor (110) ferner dazu ausgelegt ist: ein viertes Lichtsignal zu erhalten, indem der Mittelwert der Lichtsignale aus der Vielzahl von PDs berechnet wird, und die Herzfrequenzeigenschaft, die Blutsauerstoffeigenschaft und/oder die Atemfrequenzeigenschaft aus dem vierten Lichtsignal zu ermitteln; oder
der Prozessor (110) ferner dazu ausgelegt ist: ein fünftes Lichtsignal durch gewichtete Berechnung basierend auf den Lichtsignalen der Vielzahl von PDs und dem Gewicht jeder PD zu erhalten und die Herzfrequenzeigenschaft, die Blutsauerstoffeigenschaft und/oder die Atemfrequenzeigenschaft basierend auf dem fünften Lichtsignal zu ermitteln; wobei das Gewicht jeder PD durch den Prozessor (110) basierend auf der Anzahl der Male bestimmt wird, in denen die von jeder PD abgegebenen Lichtsignale in einer historischen Aufzeichnung als Grundlage für die Ermittlung verwendet werden, oder das Gewicht jeder PD ist voreingestellt.

3. Das tragbare Gerät gemäß Anspruch 1, wobei das PPG-Modul (305) zusätzlich über Schnelllade-Pins verfügt; die Schnelllade-Pins sind außerhalb der Vielzahl von PDs angebracht; und die Schnelllade-Pins sind dazu konfiguriert, eine Schnelllade-Schnittstelle für das tragbare Gerät bereitzustellen.

4. Das tragbare Gerät gemäß Anspruch 1, wobei die Anzahl der LEDs zwei beträgt; die Anzahl der PDs beträgt acht; und die acht PDs sind in einer gleichmäßigen Acht-Verteilung angeordnet, wobei der Mittelpunkt einer Verbindungslinie der beiden LEDs als Kreismittelpunkt dient.

5. Das tragbare Gerät gemäß Anspruch 1, wobei die Struktur des PPG-Moduls (305) eine konzentrische Kreisstruktur oder eine konzentrische Quadratstruktur ist.

6. Ein Steuerverfahren, wobei das Verfahren auf das tragbare Gerät gemäß einem der Ansprüche 1 bis 5 angewendet wird und das Verfahren Folgendes umfasst:
Empfangen von Lichtsignalen von einer Vielzahl von PDs; und
Ermitteln eines Herzfrequenz-Merkmals, eines Blutsauerstoff-Merkmals und/oder eines Atemfrequenz-Merkmals basierend auf den Lichtsignalen, die von der Vielzahl von PDs empfangen werden, wobei jeder PD so konfiguriert ist: Lichtsignale, die von einer LED übertragen werden, zu empfangen und die Lichtsignale an einen Prozessor (110) zu übertragen; und die Lichtsignale umfassen ein grünes Lichtsignal, ein rotes Lichtsignal und/oder ein Infrarot-Lichtsignal;
wobei das Verfahren ferner umfasst:
Steuern der LED, sodass sie ein grünes Lichtsignal und/oder ein rotes Lichtsignal aussendet, wenn festgestellt wird, dass die Umgebungslichtintensität größer oder gleich einem Lichtintensitätsschwellenwert ist;
Steuern der LED, sodass sie ein Infrarot-Lichtsignal aussendet, wenn festgestellt wird, dass die Umgebungslichtintensität kleiner als der Lichtintensitätsschwellenwert ist; und
Steuern der Frequenz des von der LED ausgesendeten Lichtsignals, wenn festgestellt wird, dass sich das tragbare Gerät in einem Bewegungszustand befindet.

7. Das Verfahren gemäß Anspruch 6, wobei das Ermitteln eines Herzfrequenz-Merkmals, eines Blutsauerstoff-Merkmals und/oder eines Atemfrequenz-Merkmals basierend auf den von der Vielzahl von PDs empfangenen Lichtsignalen umfasst:
Ermitteln eines dritten Lichtsignals mit der stärksten Signalintensität aus den Lichtsignalen der Vielzahl von PDs und Ermitteln des Herzfrequenz-Merkmals, des Blutsauerstoff-Merkmals und/oder des Atemfrequenz-Merkmals aus dem dritten Lichtsignal; oder
Ermitteln eines vierten Lichtsignals durch Berechnung eines Durchschnittswerts der Lichtsignale der Vielzahl von PDs und Ermitteln des Herzfrequenz-Merkmals, des Blutsauerstoff-Merkmals und/oder des Atemfrequenz-Merkmals aus dem vierten Lichtsignal; oder
Erhalten eines fünften Lichtsensorsignals durch gewichtete Berechnung basierend auf den Lichtsensor-Signalen von mehreren PDs und einem Gewicht für jeden PD sowie Ermittlung der Herzfrequenz-, Blutsauerstoff- und/oder Atemfrequenzmerkmale basierend auf dem fünften Lichtsensorignal; wobei das Gewicht jedes PDs vom Prozessor (110) basierend auf der Anzahl der Male bestimmt wird, die die von jedem PD emittierten Lichtsensor-Signale als Grundlage für die Erfassung in einer Historie verwendet werden, oder das Gewicht jedes PDs voreingestellt ist.

## Revendications

1. Un dispositif portable basé sur la photopléthysmographie (PPG), dans lequel le dispositif portable comprend un module PPG (305) et un processeur (110) ; le module PPG (305) comprend une pluralité de diodes électroluminescentes (LED) et une pluralité de photodiodes (PD) ;
la pluralité de PD sont réparties autour de la pluralité de LED dans une structure périphérique ;
chacune des LED est conçue pour émettre des signaux lumineux ; et les signaux lumineux comprennent : un signal de lumière verte, un signal de lumière rouge et/ou un signal de lumière infrarouge ;
chaque PD est configurée pour : recevoir les signaux lumineux, et transmettre les signaux lumineux au processeur (110) ; et
le processeur (110) est configuré pour obtenir une caractéristique de fréquence cardiaque, une caractéristique d'oxygène dans le sang et/ou une caractéristique de fréquence respiratoire à partir des signaux lumineux reçus de la pluralité de PD ;
**caractérisé en ce que** la LED est une LED intégrée tricolore dans laquelle la lumière rouge, la lumière verte et la lumière infrarouge sont combinées et **en ce que** le processeur (110) est en outre configuré pour :
contrôler la LED pour émettre un signal lumineux vert et/ou un signal lumineux rouge lorsqu'il est détecté que l'intensité lumineuse ambiante est supérieure ou égale à un seuil d'intensité lumineuse ;
contrôler la LED pour émettre un signal lumineux infrarouge lorsqu'il est détecté que l'intensité lumineuse ambiante est inférieure au seuil d'intensité lumineuse ; et
contrôler la fréquence du signal lumineux émis par la LED lorsqu'il est détecté que le dispositif portable est en état de mouvement.

2. Le dispositif portable selon la revendication 1, dans lequel le processeur (110) est en outre configuré pour : obtenir un troisième signal lumineux présentant l'intensité de signal la plus forte parmi les signaux lumineux provenant de la pluralité de PD, et obtenir la caractéristique de fréquence cardiaque, la caractéristique d'oxygène dans le sang et/ou la caractéristique de fréquence respiratoire à partir du troisième signal lumineux ; ou
le processeur (110) est en outre configuré pour : obtenir un quatrième signal lumineux en calculant une valeur moyenne des signaux lumineux provenant de la pluralité de PD, et obtenir la caractéristique de fréquence cardiaque, la caractéristique d'oxygène dans le sang et/ou la caractéristique de fréquence respiratoire à partir du quatrième signal lumineux ; ou
le processeur (110) est en outre configuré pour : obtenir un cinquième signal lumineux par calcul pondéré basé sur les signaux lumineux provenant de la pluralité de PD et un poids de chaque PD, et obtenir la caractéristique de fréquence cardiaque, la caractéristique d'oxygène dans le sang et/ou la caractéristique de fréquence respiratoire sur la base du cinquième signal lumineux ; où le poids de chaque PD est déterminé par le processeur (110) en fonction du nombre de fois où les signaux lumineux émis par chaque PD sont utilisés comme base d'obtention dans un historique, ou le poids de chaque PD est prédéfini.

3. Le dispositif portable selon la revendication 1, dans lequel le module PPG (305) comprend en outre des broches de charge rapide ; les broches de charge rapide sont disposées à l'extérieur de la pluralité de PD ; et les broches de charge rapide sont conçues pour fournir une interface de charge rapide au dispositif portable.

4. Le dispositif portable selon la revendication 1, dans lequel le nombre de LED est de deux ; le nombre de PD est de huit ; et les huit PD sont agencés selon une distribution en huit parties égales avec le point médian de la ligne de connexion des deux LED comme centre du cercle.

5. Le dispositif portable selon la revendication 1, dans lequel la structure du module PPG (305) est une structure circulaire concentrique ou une structure carrée concentrique.

6. Un procédé de contrôle, où le procédé est appliqué au dispositif portable selon l'une quelconque des revendications 1 à 5, et le procédé comprend :
recevoir des signaux lumineux provenant d'une pluralité de PD ; et
obtenir une caractéristique de fréquence cardiaque, une caractéristique d'oxygène sanguin et/ou une caractéristique de fréquence respiratoire sur la base des signaux lumineux reçus de la pluralité de PD, chaque PD étant conçu pour : recevoir les signaux lumineux transmis par une LED, et transmettre les signaux lumineux à un processeur (110) ; et les signaux lumineux comprennent un signal lumineux vert, un signal lumineux rouge et/ou un signal lumineux infrarouge ;
où le procédé comprend en outre :
contrôler la LED pour émettre un signal lumineux vert et/ou un signal lumineux rouge lorsqu'il est détecté que l'intensité lumineuse ambiante est supérieure ou égale au seuil d'intensité lumineuse ;
contrôler la LED pour émettre un signal lumineux infrarouge lorsqu'il est détecté que l'intensité lumineuse ambiante est inférieure au seuil d'intensité lumineuse ; et
contrôler la fréquence du signal lumineux émis par la LED lorsqu'il est détecté que le dispositif portable est en état de mouvement.

7. Le procédé selon la revendication 6, dans lequel obtenir une caractéristique de fréquence cardiaque, une caractéristique d'oxygène sanguin et/ou une caractéristique de fréquence respiratoire sur la base des signaux lumineux reçus de la pluralité de PD comprend :
obtenir un troisième signal lumineux présentant la plus forte intensité parmi les signaux lumineux en provenance de la pluralité de PD, et obtenir la caractéristique de fréquence cardiaque, la caractéristique d'oxygène sanguin et/ou la caractéristique de fréquence respiratoire à partir du troisième signal lumineux ; ou
obtenir un quatrième signal lumineux en calculant une valeur moyenne des signaux lumineux de la pluralité de PD, et obtenir la caractéristique de fréquence cardiaque, la caractéristique d'oxygène sanguin et/ou la caractéristique de fréquence respiratoire à partir du quatrième signal lumineux ; ou
obtention d'un cinquième signal lumineux par un calcul de pondération basé sur les signaux lumineux provenant de la pluralité de PD et sur un poids attribué à chaque PD, puis extraction de la caractéristique de rythme cardiaque, de la caractéristique de saturation en oxygène du sang et/ou de la caractéristique de fréquence respiratoire sur la base du cinquième signal lumineux ; le poids de chaque PD étant déterminé par le processeur (110) en fonction du nombre de fois où les signaux lumineux émis par chaque PD sont utilisés comme base d'obtention dans un historique, ou bien le poids de chaque PD est prédéfini.
